# EUROPEAN PATENT SPECIFICATION

(11) **EP 1 476 123 B1**
(45) Date of publication and mention of the grant of the patent: **05.04.2006**
(21) Application number: 03702731.5
(22) Date of filing: 04.02.2003
(51) Int. Cl.: A61K 8/68, A61Q 5/00, A61Q 5/06

(54) **USE OF CERAMIDES AND SIMILAR COMPOUNDS IN HAIR CARE COMPOSITIONS**
VERWENDUNG VON CERAMIDEN IN HAARBEHANDLUNGSMITTELN
UTILISATION DE CERAMIDES ET DE COMPOSES SIMILAIRES DANS DES COMPOSITIONS DE SOINS CAPILLAIRES

(30) Priority: 22.02.2002 GB 0204133
(43) Date of publication of application: 17.11.2004
(73) Proprietor: Quest International Services B.V., 1411 GP Naarden (NL)
(72) Inventor: WATKINS, Stephen, Ashford, Kent TN24 9HZ (GB); EYRE, Heather, Canterbury, Kent CT 11XQ (GB)
(74) Representative: Matthews, Heather Clare
(86) International application number: PCT/GB2003/000483
(87) International publication number: WO 2003/070209

(56) References cited:
- EP-A- 0 739 625
- EP-A- 1 036 556
- GB-A- 2 307 407
- US-A- 5 476 661
- US-A- 5 700 456
- US-A- 6 039 962
- US-A- 6 076 530
- DATABASE CA [Online] CHEMICAL ABSTRACTS SERVICE, COLUMBUS, OHIO, US; EYRE, HEATHER ET AL: "The effect of ceramide-related ingredients on the deposition of hair dyes" retrieved from STN Database accession no. 137:98611 XP002238525 & FRAGRANCE JOURNAL (2002), 30(6), 54-59 ,

## Description

This concerns hair care compositions, particularly non-oxidative hair colouring compositions, and methods of improving the colour effects including the colour-fastness of non-oxidative hair colouring compositions.

### Background to the Invention

Compositions for colouring or dyeing hair can be categorised into temporary, semi-temporary, semi-permanent, permanent and, more recently, the so-called demi-permanent products. Although these names try to describe the general longevity of the colour in terms of wash-out, there is considerable blurring of the categories depending upon the brand of hair colour purchased, the dyes used and the formulation of the carrier base. However, as a guide:
- Temporary lasts only one wash
- Semi-temporary lasts approximately 5 washes
- Semi-permanent lasts approximately 10 washes
- Demi-permanent last approximately 20 washes
- Permanent is not washed out and lasts until the colour fades or grows out

Other products on the market are sold as post-colour formulations and these claim to extend the life of the hair colour. These can be split into 'colour maintenance' products, normally shampoos, which are formulated to be mild and help reduce colour washout, and 're-tint' products, which possess a low concentration of colour to reinforce the existing colour of the hair.

The chemistry of hair dyes differs greatly. Temporary and semi-temporary colours tend to be larger molecules that are unable to penetrate the hair shaft and so reside on the hair surface. This makes them easy to remove with washing. Semi-permanent, demi-permanent and permanent colours are smaller molecules, which are able to penetrate the hair shaft, making them more resistant to hair washing.

The following list is a guide to the types of dye often found in the particular category of hair product. It should be noted that many commercially available products contain combinations of dye types:
- Temporary hair colours include food or vegetable dyes or their insoluble lakes (pigments). Here, the colouring formulation normally contains fixatives such as resins to hold the colour onto the hair. These are easily removed with shampoo.
- Semi-temporary dyes are cationic in charge. The cationic charge allows them to bind to the hair surface and makes them more resistant to washing.
- Semi-permanent dyes are normally small, nitro-dyes, which are able to penetrate the hair shaft. This makes them less readily removed with washing.
- Permanent dyes (and couplers) are themselves colourless precursors called oxidation dyes. These precursors, in the presence of peroxide, undergo a chemical reaction to produce coloured compounds of varying degrees of polymerisation. Penetration of the colourless precursors into the hair fibre can be 'encouraged' by the use of an alkali, such as ammonia, which causes the hair shaft to swell and become more porous. Once within the hair fibre, the small colourless precursors polymerize to form the larger coloured compounds. These are then trapped within the hair fibre by their physical size.
- Demi-permanent colours use similar, colourless, precursors to the permanent colours. However, the use of different coupling agents restricts polymerization to the formation of smaller coloured compounds. These smaller compounds are more easily washed out of the hair. Also, the reduction or elimination of ammonia from the formulation helps to limit the penetration of the precursors.

Many products contain a combination of different dye types. For example, some semi-temporary dyes are mixed with the semi-permanent dyes to produce more natural looking shades. The demi-permanent and permanent products can provide a wide variety of colours even on dark hair because the natural hair colour is bleached during the process. The use of the temporary, semi-temporary and semi-permanent colours are dependent upon the individual's natural hair colour; a dark haired person cannot become blond.

The reason for the recent popularity in demi-permanent colouration comes from the desire to produce a more natural look and reduce damage to the hair. Demi-permanent colouring is also used to boost an old, fading, permanent colour.

Semi-temporary or semi-permanent products are used to tint the hair. A growing market for these colours is also in masking grey hair. This is because these types of colours effect the lighter (white) hairs more than the darker hairs, helping to provide a more natural look. However, as described above, such products do not last very long.

Ceramides are a group of naturally occurring lipids present in the stratum corneum of the skin and the cuticle of the hair. The structures of ceramides are described by Wertz P. W. , Miethke M.C., Long S.A., Strauss J.M. and Downing D.T. in *'The composition of ceranzides from human stratum corneum and from comedones',* The Journal of Investigative Dermatology, 84, 41-412 (1985). Ceramide structures have been classified into 6 families known as ceramide 1 to ceramide 6 (with the ceramide 6 family being subclassified into 2 members, ceramide 6i and ceramide 6ii). These families are described by Downing in Arch. Dermatol., Vol 123, 1381-1384, 1987. Ceramides in each family can vary in chain length and unsaturation.

Ceramides are known to have beneficial effects on skin and hair, and it is known to use ceramides and functionally similar related materials (including glycoceramides, ceramide derivatives, ceramide analogues, neoceramides and so-called pseudoceramides (which are non naturally occurring variants of ceramides)), either naturally occurring or synthetically produced, in skin-care and hair-care compositions.

US 6251378 of L'Oreal concerns use of ceramide compounds in oxidation dyeing processes for decreasing the degradation of colour of dyed keratin fibres, particularly human hair. Oxidation dyeing processes generally result in degradation of keratin fibres, and the ceramide compounds act to protect fibres from damage during the oxidation dyeing process so they remain softer and less brittle (see column 2, lines 38 to 42).

US 6110450 of Helene Curtis concerns hair care compositions, particularly shampoos and conditioners, including at least one ceramide and/or glycoceramide and phytantriol, which are said to provide hair conditioning benefits. There is incidental, passing reference to the possibility of using such compositions in conjunction with hair dyes (oxidation dyes and/or direct dyes) for conditioning benefits, but no examples are included.

### Summary of the Invention

It has surprisingly been found that ceramides and functionally similar related materials have the effect of enhancing the colouring effects of nqn-oxidative dyes and also enhancing the colour-fastness of hair dyed or coloured by non-oxidative methods. This effect is neither disclosed nor suggested in the prior art. Furthermore this effect is not predictable from US 6251378 as non-oxidative dyes do not damage keratin fibres so the protective effect of ceramide compounds disclosed in US 6251378 will not arise for non-oxidative processes and compositions.

In one aspect the invention provides a method of improving the colouring effects of non-oxidative hair colouring compositions, comprising application to the hair (during or after dyeing) of ceramide or functionally similar related material as defined hereinafter, in the absence of phytantriol.

The invention also includes within its scope use of ceramide or functionally similar related material as defined below in a hair care composition for the purpose of improving the colouring effects of a non-oxidative hair dye.

The expression "ceramide or functionally similar related material" is used to mean ceramides, glycoceramides, pseudoceramide and neoceramides. The materials may be naturally occurring or synthetically produced. In general terms, such materials comprise two fatty chains attached to a polar head group. Each fatty chain can vary in length from C10 to C30 but will typically be between C12 and C24 and even more typically between C14 and C18. The degree of saturation and/or branching of the claims can also vary. The fatty chains may also contain one or more functional side groups; normally, but not exclusively, alcohol groups. The fatty chains can be acids, alcohols, amines or amides. Each fatty chain can be joined to the polar head group through an ester, ether, amine or amide bond. The polar head group consists of the functional groups associated with the fatty chains and one or more additional functional groups, normally alcohol(s), which may be further modified with polyols, ethylene oxide, etc.

Suitable materials are well known to those skilled in the art and are disclosed e.g. in US 6251378, US 6110450, US 6077972, EP 482860, EP 97059 and WO 94/07844 and many other documents. Currently preferred materials include the pseudoceramide bishydroxyethyl biscetyl malonamide (a lipid with ceramide functionality) as described in Example 1 of WO 94/07844 and as available from Quest under the Trade Mark Questamide H; ceramide 2, particularly in the nature-identical form obtained by synthesis and as available from Quest under the Trade Mark Ceramide II; and a blend of lipids with the pseudoceramide bishydroxyethyl biscetyl malonamide (Questamide H) and other components found naturally in the skin and hair such as sterol, fatty acids, phospholipids and glycerine, as available from Quest under the Trade Mark Questamix H.

Mixtures of two or more ceramides or functionally similar related materials may be used.

The invention can thus be put into effect by incorporating ceramide or functionally similar related material into a hair care composition for use during non-oxidative dyeing of hair (i.e. a non-oxidative hair colouring composition) where it acts to improve dye deposition onto hair, or into a composition for use on hair after dyeing by a non-oxidative method, e.g. a shampoo or conditioner composition, to help maintain the colour of dyed hair.

The invention improves the colouring effect of non-oxidative dyes by two effects, namely:
i) by producing improved deposition of colour onto hair when used at the time of colouring treatment (e.g. by being incorporated into a non-oxidative hair colouring composition); and
ii) by reduced colour loss on washing of dyed hair (e.g. by being incorporated into a shampoo), resulting in better maintenance of colour, i.e. improving colour fastness. The invention can thus enhance the effectiveness and the effective life of non-oxidative hair colouring compositions.

Ceramides or functionally similar related material is suitably present in a hair care composition in an amount in the range 0.01 to 5% by weight, preferably 0.05 to 1% by weight. Very effective results have been obtained with non-oxidative hair colouring compositions and shampoos containing 0.1 % by weight Questamide H, with good results being obtained with compositions containing 0.5% by weight Questamix H or 0.05% by weight Ceramide II.

The hair care compositions can otherwise be of generally conventional formulation.

The invention will be further described, by way of illustration, in the following Examples.

### Example 1 - Semi-temporary hair colour maintenance

Three hair swatches were coloured with the same colorant conditioner (the formula of which is given below). The three swatches were temporarily put together to form a single tress and then wetted with water at 45°C. Seventy grams of colour conditioner was gently massaged into the hair tress for approximately 4 minutes, ensuring that the tress was completely coated. The colour was then left in the hair for 15 minutes before being rinsed with running water, at 45°C, until the water ran clear (circa 3 minutes). Finally the tress was dried with a hair-dryer (with gentle combing).

| Conditioner formula: | | |
|---|---|---|
| | | %w/w |
| | Propylene glycol | 17 % |
| | Hydroxyethylcellulose¹ | 0.6% |
| | Phenonip² | 0.9 % |
| | Water | 51.7 % |
| | | |
| | Arlacel 165³ | 5 % |
| | Cetyl stearyl alcohol⁴ | 3 % |
| | C12-15 Alkyl Benzoate⁵ | 0.5% |
| | Cetearyl Isononanoate⁶ | 0.5 % |
| | Lauryl Lactate⁷ | 0.5 % |
| | | |
| | Water | 20 % |
| | Colour blend | 0.2 % |
| | | |
| | Citric acid | to pH3 |
| | | |
| The colour blend contains: | CI 56059 is Basic Blue 99 | |
| | CI 12245 is Basic Red 76 | |
| Ratio red/blue: 40:60 | | |

| | | |
|---|---|---|
| 1. available as Natrosol 250HHR from Aqualon/Hercules, Inc. | | |
| 2. Phenoxyethanol (and) methylparaben (and) ethylparaben (and) propylparaben (and) butylparaben available from Nipa Labs. | | |
| 3. Glyceryl Stearate (and) PEG-100 Stearate, available from Uniqema. | | |
| 4. available as Lanette O from Cognis. | | |
| 5. available as Finsolv TN from Finetex, Inc. | | |
| 6. available as Cetiol SN from Cognis. | | |
| 7. available as Crodamol LL from Croda Oleochemicals Limited. | | |

The hair tress was then separated again into three swatches. Each swatch was then cut into two parts in order to obtain six smaller hair swatches, all evenly coloured. Two swatches were kept for colour reference and labelled "initial colour". The remaining four swatches were then each washed with a different shampoo containing a selected Quest ingredient and labelled Xn (where X is the formula and n is the number of washes).

Four different formulae of shampoo were made:

| **FORMULA A** | |
|---|---|
| | % w/w |
| Sodium laureth sulfate⁸ | 35 % |
| Cocamidopropyl betaine⁹ | 10% |
| Cocamide DEA¹⁰ | 2 % |
| Sodium chloride | 2.1% |
| Questamide H | 0.1% |
| Water | 50.8% |

| **FORMULA B** | |
|---|---|
| | %w/w |
| Sodium laureth sulfate⁸ | 35 % |
| Cocamidopropyl betaine⁹ | 10% |
| Sodium chloride | 2.1% |
| Water | 50.9% |

| **FORMULA C** | |
|---|---|
| | %w/w |
| Sodium laureth sulfate⁸ | 35% |
| Cocamidopropyl betaine⁹ | 10% |
| Cocamide DEA¹⁰ | 2 % |
| Sodium chloride | 2.1% |
| Questamix H | 0.5% |
| Water | 50.4% |

| **FORMULA D** | |
|---|---|
| | % w/w |
| Sodium laureth sulfate⁸ | 35 % |
| Cocamidopropyl betaine⁹ | 10% |
| Cocamide DEA¹⁰ | 2% |
| Sodium chloride | 2.1 % |
| Ceramide II | 0.05 % |
| Water | 50.85% |

| | |
|---|---|
| 8. available as Texapon NSO/IS from Cognis. | |
| 9. available as Tegobetain F50 from Th Goldschmidt AG. | |
| 10. available as Empilan CDE from Cognis. | |

The four hair swatches were each washed seven times following a precise protocol, with each swatch being shampooed in a different shampoo (A, B, C or D). The swatches were firstly wetted with water at 45°C. Each swatch was 'coated' in 10 grams of the relevant shampoo and then, whilst being supported in one hand, rubbed with the fingers in a way that best reproduces the action of washing the hair *in vivo.* The hair was rinsed clean with running water at 45°C before being dried with a hair dryer (with gentle combing).

Using a Minolta Colorimeter, the base hair swatch colour was measured prior to application of the colour, after the hair had been coloured, and after the final (seventh) wash and dry regime. The freshly coloured swatches and the final washed/dried swatches were also visually compared and ranked (blind) for colour intensity, using an untrained panel of volunteers.

The Minolta Colorimeter measures three aspects of colour:
**L** is the luminosity (0 black and 100 very bright)
**a** is a green-red scale (negative is green and positive is red)
**b** is a blue-yellow scale (negative is blue and positive is yellow)

### Colorimeter Results

| Hair Swatch Colour Before Colouring: | | | | | | | |
|---|---|---|---|---|---|---|---|
| ① | L 78.62 | ② | L 79.23 | ③ | L 78.60 | **mean** | **L 78.82** |
| | a +2.71 | | a + 1.89 | | a +2.14 | | **a +2.25** |
| | b +20.79 | | b +20.08 | | b +20.40 | | **b +20.42** |

| "Initial Colour" Swatches (not washed): | | | | | | | |
|---|---|---|---|---|---|---|---|
| ① | L 25.25 | ② | L 28.82 | ③ | L 30.41 | **mean** | **L 28.16** |
| | a +12.34 | | a + 13.31 | | a + 13.23 | | **a + 12.96** |
| | b -0.69 | | b -1.45 | | b -0.50 | | **b -0.88** |

| Xn Swatches After Seven Washes: | | | | |
|---|---|---|---|---|
| | **SWATCH** | **SWATCH** | **SWATCH** | **SWATCH** |
| | **A**_{**7**} | **B**_{**7**} | **C**_{**7**} | **D**_{**7**} |
| **Measure 1** | L 45.83 | L 51.11 | L 49.62 | L 47.20 |
| | a +9.36 | a +9.37 | a 8.90 | a + 10.38 |
| | b+2.11 | b+2.29 | b+3.42 | b+1.62 |
| **Measure 2** | L 50.39 | L 52.58 | L 52.44 | L 48.52 |
| | a +7.66 | a +7.91 | a +8.43 | a +9.93 |
| | b +3.41 | b +2.95 | b +3.16 | b +2.75 |
| **Measure 3** | L 50.10 | L 55.98 | L 51.14 | L 54.08 |
| | a +9.18 | a +7.53 | a +9.97 | a +7.68 |
| | b +3.02 | b 4.07 | b +3.50 | b +3.24 |
| **Mean** | **L 48.78** | **L 53.22** | **L 51.06** | **L 49.93** |
| | **a +8.73** | **a +8.27** | **a +9.1** | **a +9.33** |
| | **b +2.85** | **b +3.10** | **b +3.36** | **b +2.53** |

The colour difference between the freshly coloured hair and the final washed/dried hair (final hair colour - initial hair colour) can be calculated from the Minolta Colorimeter measurements. This gives Δ L, Δ a and Δ b for each formulation. It should be noted that a smaller Δ L value represents a darker hair swatch (more hair colour), a more negative Δ a value represents less red (less hair colour), and a more positive Δ b value represents less blue (less hair colour):

| | Δ **L** | Δ **a** | Δ **b** |
|---|---|---|---|
| Questamide H (formula A) | +20.62 | -4.23 | +3.73 |
| Questamix H (formula C) | +22.90 | -3.86 | +4.24 |
| Ceramide II (formula D) | +21.77 | -3.63 | +3.41 |
| **Without ingredient (formula B)** | **+25.06** | **-4.69** | **+3.98** |

### Visual Assessment (ranking)

| **After 7 washes** | **Stronger colour** | | | **Less coloured** |
|---|---|---|---|---|
| Volunteer 1 | A | D | C | B |
| Volunteer 2 | A | D | C | B |
| Volunteer 3 | D | A | C | B |
| Volunteer 4 | A | C | D | B |
| Volunteer 5 | A | D | C | B |

### Analysis of Data

The Δ L, Δ a and Δ b values show that the best colour retention was obtained with Questamide H, followed by Ceramide II/Questamix H. The base shampoo performed the worst.

Visual assessment (ranking) of the colour of the hair swatches confirmed that the Questamide H containing shampoo provided the best colour retention. Next came Ceramide II, then Questamix H and, lastly, the base shampoo.

### Conclusion

The base shampoo, which was formulated using the actives found in most shampoos on the market, was shown to cause a high loss of temporary hair colour over the seven washes. Incorporation of the Quest ceramide ingredients into the shampoo base reduced the amount of colour lost.

### Example 2 - Semi-temporary hair colour deposition and maintenance

Four hair swatches, each 21 cm long and weighing 13 grams, were coloured with different formulae containing selected Quest ingredients following a precise protocol:

| **FORMULA E** | |
|---|---|
| | %w/w |
| Propylene glycol | 17% |
| Natrosol 250HHR | 0.6 % |
| Phenonip | 0.9 % |
| Water | 51.7 % |
| Arlacel 165 | 5 % |
| Cetyl stearyl alcohol | 3 % |
| Finsolv TN | 0.5 % |
| Cetiol SN | 0.5 % |
| Crodamol LL | 0.5 % |
| Questamide H | 0.1 % |
| Water | 20 % |
| Colour blend | 0.2 % |
| Citric acid | to pH3 |

| **FORMULA F** | |
|---|---|
| | %w/w |
| Propylene glycol | 17% |
| Natrosol 250HHR | 0.6 % |
| Phenonip | 0.9 % |
| Water | 51.8% |
| Arlacel 165 | 5 % |
| Cetyl stearyl alcohol | 3 % |
| Finsolv TN | 0.5 % |
| Cetiol SN | 0.5 % |
| Crodamol LL | 0.5 % |
| Water | 20% |
| Colour blend | 0.2% |
| Citric acid | to pH3 |

| **FORMULA G** | |
|---|---|
| | %w/w |
| Propylene glycol | 17% |
| Natrosol 250HHR | 0.6% |
| Phenonip | 0.9% |
| Water | 51.3 % |
| Arlacel 165 | 5 % |
| Cetyl stearyl alcohol | 3 % |
| Finsolv TN | 0.5 % |
| Cetiol SN | 0.5 % |
| Crodamol LL | 0.5 % |
| Questamix H | 0.5 % |
| Water | 20% |
| Colour blend | 0.2% |
| Citric acid | to pH3 |

| **FORMULA H** | |
|---|---|
| | %w/w |
| Propylene glycol | 17 % |
| Natrosol 250HHR | 0.6 % |
| Phenonip | 0.9 % |
| | |
| Water | 51.75 % |
| Arlacel 165 | 5% |
| Cetyl stearyl alcohol | 3 % |
| Finsolv TN | 0.5% |
| Cetiol SN | 0.5% |
| Crodamol LL | 0.5% |
| Ceramide II | 0.05% |
| Water | 20% |
| Colour blend | 0.2% |
| Citric acid | to pH3 |

The colour blend contains:
CI 56059 is Basic Blue 99 and
CI 12245 is Basic Red 76
Ratio red/blue: 40:60

The hair swatches were first wetted with water at 45°C. Thirty grams of each colour conditioner formula was applied to a single wetted hair swatch and massaged gently into the hair for 3 minutes using a gloved hand. These colour conditioners were then left on the hair for 15 minutes before being rinsed with running water, at 45°C, until the water ran clear (circa 2 minutes). Finally the swatches were dried using a 500 Watt hair-dryer with gentle combing.

### Washing

Each coloured hair swatch was cut into 2 equal parts: one to keep as the original colour (labelled X₀ where X is the formula reference) and the other to test the colour resistance to repeated shampooing (labelled Xₙ where X is the formula reference and n represents the number of washes). The swatches were each washed seven times following a precise protocol. The swatches were firstly wetted with water at 45°C. Each swatch was 'coated' in 10 grams of shampoo formula below and then, whilst being supported in one hand, rubbed with the fingers in a way that best reproduces the action of washing the hair *in vivo.* The swatches were rinsed clean with running water at 45°C before being dried with a hair dryer (with gentle combing).

**Shampoo formula used for the test:**

| | % w/w |
|---|---|
| Sodium laureth (2) sulfate (28%) | 12.00 |
| Cocamidopropyl betaine (28%) | 10.00 |
| Cocamide DEA | 2.00 |
| Sodium chloride | 2.10 |
| Preservative | 0.10 |
| Water | 73.80 |

### Colour analysis

Using a Minolta Colorimeter, the base hair swatch colour was measured prior to application of the colour, after the hair had been coloured, and after each washing/drying regime. The freshly coloured swatches and the final washed/dried swatches were also visually compared and ranked (blind) for colour intensity, using an untrained panel of volunteers.

### Results

### Colorimeter measurements

| Hair Swatch Colour before colouring: | | | | | | | |
|---|---|---|---|---|---|---|---|
| ① | L 78.62 | ② | L 79.23 | ③ | L 78.60 | **mean** | **L 78.82** |
| | a +2.71 | | a +1.89 | | a +2.14 | | **a +2.25** |
| | b +20.79 | | b +20.08 | | b +20.40 | | **b +20.42** |

| After colouring (not washed): | | | | |
|---|---|---|---|---|
| | **SWATCH E**_{**0**} | **SWATCH F**_{**0**} | **SWATCH G**_{**0**} | **SWATCH H**_{**0**} |
| **Measure 1** | L 27.81 | L 26.77 | L 31.68 | L 27.02 |
| | a +12.92 | a +12.58 | a +13.71 | a +12.14 |
| | b -1.62 | b -0.23 | b -1.33 | b -1.32 |
| **Measure 2** | L 27.13 | L 31.13 | L 29.73 | L 28.74 |
| | a + 12.09 | a + 14.25 | a + 13.87 | a + 12.06 |
| | b -1.69 | b -1.13 | b -1.55 | b -1.78 |
| **Measure 3** | L 28.18 | L 29.89 | L 30.74 | L 29.10 |
| | a + 12.24 | a + 13.34 | a + 12.79 | a + 12.90 |
| | b-1.81 | b-0.60 | b-0.81 | b-1.78 |
| **Mean** | **L 27.70** | **L 29.3** | **L 30.72** | **L 28.3** |
| | **a + 12.42** | **a + 13.4** | **a + 13.45** | **a + 12.4** |
| | **b -1.70** | **b -0.65** | **b -1.23** | **b -1.6** |

| After the first wash: | | | | |
|---|---|---|---|---|
| | **SWATCH E**_{**1**} | **SWATCH F**_{**1**} | **SWATCH G**_{**1**} | **SWATCH H**_{**1**} |
| **Measure 1** | L 35.89 | L 40.11 | L 34.28 | L 34.93 |
| | a +12.15 | a +12.35 | a + 13.20 | a +12.82 |
| | b -1.57 | b +1.49 | b -1.28 | b -2.46 |
| **Measure 2** | L 37.22 | L 34.19 | L 35.37 | L 32.29 |
| | a +11.99 | a +13.17 | a +12.77 | a +13.14 |
| | b -0.99 | b -0.51 | b -0.96 | b -1.86 |
| **Measure 3** | L 32.89 | L 32.12 | L 36.84 | L 34.07 |
| | a + 12.85 | a + 12.54 | a +12.43 | a +13.18 |
| | b -1.79 | b -0.62 | b -0.65 | b -1.75 |
| **Mean** | **L 35.33** | **L 35.47** | **L 35.50** | **L 33.76** |
| | **a + 12.33** | **a + 12.72** | **a + 12.81** | **a + 13.05** |
| | **b -1.45** | **b + 0.12** | **b -0.96** | **b -2.02** |

| After the second wash: | | | | |
|---|---|---|---|---|
| | **SWATCH E**_{**2**} | **SWATCH F**_{**2**} | **SWATCH G**_{**2**} | **SWATCH H**_{**2**} |
| **Measure 1** | L 43.79 | L 38.93 | L 38.25 | L 38.54 |
| | a + 10.84 | a + 12.24 | a + 13.97 | a + 12.44 |
| | b -0.34 | b +0.24 | b -0.56 | b -0.95 |
| **Measure 2** | L 35.44 | L 38.85 | L 41.77 | L 41.69 |
| | a + 13.35 | a +11.80 | a +12.63 | a +11.39 |
| | b -2.15 | b +0.37 | b +0.52 | b -0.39 |
| **Measure 3** | L 39.19 | L 43.77 | L 40.67 | L 38.03 |
| | b -1.55 | b + 1.22 | b -0.58 | b -1.54 |
| **Mean** | **L 39.47** | **L 40.51** | **L 40.23** | **L 39.42** |
| | **a +12.62** | **a +11.81** | **a +13.29** | **a +12.44** |
| | **b -1.35** | **b + 0.61** | **b -0.21** | **b -0.94** |

| After the third wash: | | | | |
|---|---|---|---|---|
| | **SWATCH E**_{**3**} | **SWATCH F**_{**3**} | **SWATCH G**_{**3**} | **SWATCH H**_{**3**} |
| **Measure 1** | L 37.89 | L 42.86 | L 42.00 | L 42.15 |
| | a +12.77 | a +12.80 | a +12.58 | a +12.51 |
| | b -1.20 | b -0.55 | b -0.85 | b -0.85 |
| **Measure 2** | L 42.32 | L 42.04 | L 42.74 | L 40.32 |
| | a +12.77 | a +13.26 | a +13.22 | a +13.16 |
| | b -1.35 | b -0.69 | b -0.72 | b -0.79 |
| **Measure 3** | L 39.81 | L 42.22 | L 43.07 | L 41.93 |
| | a + 13.36 | a + 13.29 | a + 13.30 | a +12.40 |
| | b -1.84 | b +0.32 | b -0.93 | b -1.09 |
| **Mean** | **L 40.00** | **L 42.37** | **L 42.60** | **L 41.46** |
| | **a +12.97** | **a +13.12** | **a +13.03** | **a +12.69** |
| | **b -1.46** | **b -0.31** | **b -0.83** | **b -0.91** |

| After the fourth wash: | | | | |
|---|---|---|---|---|
| | **SWATCH E**_{**4**} | **SWATCH F**_{**4**} | **SWATCH G**_{**4**} | **SWATCH H**_{**4**} |
| **Measure 1** | L 43.50 | L 46.87 | L 46.51 | L 43.50 |
| | a + 12.46 | a + 11.76 | a + 12.58 | a + 12.39 |
| | b -0.98 | b +0.95 | b+1.19 | b+0.06 |
| **Measure 2** | L 42.80 | L 45.40 | L 43.33 | L 45.91 |
| | a +13.13 | a +12.57 | a +13.52 | a +11.22 |
| | b -0.92 | b +0.84 | b -0.61 | b +0.17 |
| M**easure 3** | L 43.30 | L 47.00 | L 47.20 | L 44.49 |
| | a + 13.00 | a + 11.66 | a + 11.90 | a + 11. 87 |
| | b -0.17 | b +0.90 | b + 1.07 | b +0.21 |
| **Mean** | **L 43.2** | **L 46.42** | **L 45.68** | **L 44.63** |
| | **a + 12.86** | **a + 11.99** | **a + 12.66** | **a + 11.83** |
| | **b -0.69** | **b +0.90** | **b +0.55** | **b +0.15** |

| After the fifth wash: | | | | |
|---|---|---|---|---|
| | **SWATCH E**_{**5**} | **SWATCH F**_{**5**} | **SWATCH G**_{**5**} | **SWATCH H**_{**5**} |
| **Measure 1** | L 41.73 | L 50.56 | L 48.06 | L 46.61 |
| | a +12.25 | a +9.96 | a +11.19 | a +10.37 |
| | b -0.92 | b +2.32 | b +0.86 | b +0.36 |
| **Measure 2** | L 46.83 | L 45.47 | L 47.71 | L 46.43 |
| | a +11.04 | a +11.84 | a +10.79 | a +10.95 |
| | b +0.61 | b +1.71 | b +0.61 | b +0.85 |
| **Measure 3** | L 46.96 | L 51.84 | L 46.60 | L 46.06 |
| | a +10.83 | a +9.97 | a +11.35 | a +10.45 |
| | b +0.45 | b +3.17 | b +0.88 | b +1.62 |
| **Mean** | **L 45.17** | **L 49.29** | **L 47.46** | **L 46.37** |
| | **a +11.37** | **a +10.59** | **a +11.11** | **a +10.59** |
| | **b+0.05** | **b+2.40** | **b +0.78** | **b +0.94** |

| After the sixth wash: | | | | |
|---|---|---|---|---|
| | **SWATCH E**_{**6**} | **SWATCH F**_{**6**} | **SWATCH G**_{**6**} | **SWATCH H**_{**6**} |
| **Measure 1** | L 46.71 | L 52.21 | L 49.52 | L 51.24 |
| | a + 10.80 | a +9.40 | a +9.96 | a +8.34 |
| | b +0.40 | b +3.40 | b + 1.84 | b +2.46 |
| **Measure 2** | L 46.20 | L 53.14 | L 48.76 | L 45.50 |
| | a +10.85 | a +8.74 | a +10.20 | a +9.65 |
| | b -0.43 | b +3.40 | b + 1.40 | b +0.75 |
| **Measure 3** | L 46.72 | L 53.82 | L 51.53 | L 50.61 |
| | a +10.76 | a +8.94 | a +9.73 | a +8.47 |
| | b +0.32 | b +3.77 | b +2.26 | b +2.52 |
| **Mean** | **L 46.54** | **L 53.05** | **L 49.94** | **L 49.12** |
| | **a +10.80** | **a +9.03** | **a +9.96** | **a +8.82** |
| | **b +0.09** | **b +3.52** | **b +1.83** | **b +1.91** |

| After the seventh wash: | | | | |
|---|---|---|---|---|
| | **SWATCH E**_{**7**} | **SWATCH F**_{**7**} | **SWATCH G**_{**7**} | **SWATCH H**_{**7**} |
| **Measure 1** | L 48.94 | L 52.54 | L 52.74 | L 49.84 |
| | a +10.10 | a +9.14 | a +9.70 | a +10.77 |
| | b +0.68 | b +3.36 | b +2.77 | b +0.64 |
| **Measure 2** | L 47.67 | L 52.46 | L 51.32 | L 54.00 |
| | a + 10.94 | a +9.15 | a +10.69 | a +8.55 |
| | b + 1.06 | b +3.01 | b +3.58 | b +2.91 |
| **Measure 3** | L 55.20 | L 55.38 | L 50.87 | L 52.55 |
| | a +8.16 | a +9.04 | a +10.53 | a +9.35 |
| | b +3.31 | b +4.52 | b +2.70 | b +2.23 |
| **Mean** | **L 50.60** | **L 54.1** | **L 51.64** | **L 52.13** |
| | **a +9.73** | **a +9.11** | **a +10.31** | **a +9.56** |
| | **b +1.68** | **b +3.63** | **b +3.02** | **b +1.93** |

The colour difference between the freshly coloured hair and the final washed/dried hair (final hair colour - initial hair colour) can be calculated from the Minolta Colorimeter measurements. This gives Δ L, Δ a and Δ b for each formulation. It should be noted that a smaller Δ L value represents a darker hair swatch (more hair colour), a more negative Δ a value represents less red (less hair colour), and a more positive Δ b value represents less blue (less hair colour):

| | **ΔL** | **Δa** | **Δb** |
|---|---|---|---|
| Questamide H (formula E) | +22.9 | -2.69 | +3.38 |
| Questamix H (formula G) | +20.92 | -3.14 | +4.25 |
| Ceramide II (formula H) | +23.83 | -2.84 | +3.53 |
| Without ingredient (formula F) | +24.8 | -4.29 | +4.28 |

### Visual Assessment (ranking)

| **After colouring** | **Stronger colour** | | | **Less coloured** |
|---|---|---|---|---|
| Volunteer 1 | E | H | G | F |
| Volunteer 2 | G | E | H | F |
| Volunteer 3 | E | H | G | F |
| Volunteer 4 | E | H | G | F |
| Volunteer 5 | E | G | H | F |
| Volunteer 6 | E | H | G | F |
| Volunteer 7 | G | E | H | F |

| **After 7 washes** | **Stronger colour** | | | **Less coloured** |
|---|---|---|---|---|
| Volunteer 1 | E | H | G | F |
| Volunteer 2 | G | E | H | F |
| Volunteer 3 | E | G | H | F |
| Volunteer 4 | E | H | G | F |
| Volunteer 5 | E | H | G | F |
| Volunteer 6 | E | G | H | F |
| Volunteer 7 | E | G | H | F |

### Analysis of Data

### Hair Colour Prior to Washing

Comparison of the mean 'L', 'a' and 'b' values of the swatches, prior to washing, shows that the best colour deposition was obtained with the Questamide H containing formulation, followed by Ceramide II and/or Questamix H, and finally the base colour conditioner. The visual ranking of the hair swatches confirms this result, with hair swatches treated with Questamide H, Ceramide II or Questamix H showing a stronger colour intensity compared to the base conditioner treated swatch being chosen unanimously as the poorest.

### Hair Colour After Washing Seven Times

The Δ L, Δ a and Δ b values show the same ranking as that observed prior to washing. Visually, it becomes even clearer that Questamide H produces the strongest hair colouring, followed by Questamix H/Ceramide II, and then, finally, the base conditioner.

### Conclusion

The use of the Quest lipids at the above concentrations, particularly Questamide H, produces a stronger colour deposition than the base colour conditioner alone. This effect is maintained following repeated washing with shampoo.

### Example 3 - Semi-permanent hair colour deposition

Four hair swatches, each 21 cm long and weighing 13 grams, were coloured with different formulae containing selected Quest ingredients following a precise protocol:

| **FORMULA I** | |
|---|---|
| | %w/w |
| Propylene glycol | 10% |
| Natrosol 250HHR | 1.5% |
| Phenonip | 0.5% |
| Water | 84.15% |
| Plantacare 2000 UP¹ | 0.25 % |
| Cetyl alcohol | 1.5 % |
| Ceteareth-20² | 1.5 % |
| Questamide H | 0.1 % |
| Colour Base | 10 % |

| **FORMULA J** | |
|---|---|
| | %w/w |
| Propylene glycol | 10% |
| Natrosol 250HHR | 1.5 % |
| Phenonip | 0.5 % |
| Water | 84.25% |
| Plantacare 2000 UP¹ | 0.25 % |
| Cetyl alcohol | 1.5 % |
| Ceteareth-20² | 1.5% |
| | |
| Colour Base | 10 % |

| **FORMULA K** | |
|---|---|
| | %w/w |
| Propylene glycol | 10% |
| Natrosol 250HHR | 1.5% |
| Phenonip | 0.5% |
| Water | 83.75% |
| Plantacare 2000 UP¹ | 0.25% |
| Cetyl alcohol | 1.5% |
| Ceteareth-20² | 1.5% |
| Questamix H | 0.5 % |
| Colour Base | 10 % |

| **FORMULA L** | |
|---|---|
| | %w/w |
| Propylene glycol | 10% |
| Natrosol 250HHR | 1.5% |
| Phenonip | 0.5% |
| Water | 84.2% |
| Plantacare 2000 UP¹ | 0.25% |
| Cetyl alcohol | 1.5% |
| | |
| Ceteareth-20² | 1.5% |
| Ceramide II | 0.05% |
| Colour Base | 10% |

The colour base contains:

| | |
|---|---|
| Ethoxydiglycol | 5.5% |
| Water | 4.2% |
| 3-Nitro-p-hydroxyethylaminophenol | 0.3 % |

| | |
|---|---|
| 1. Sodium Laureth Sulphate (and) Lauryl Glucoside¹ available from Cognis. | |
| 2. available as Eumulgin B2 from Cognis. | |

Preparation of the conditioners was carried out by mixing all the ingredients and heating to 80°C. Once the solution had reached the desired temperature it was mixed under high shear. The solution was then cooled with continuous slow stirring. Finally 5 grams of the colour base was added to 45 grams of each of the base formulations.

The hair swatches were first wetted with water at 45°C. Thirty grams of each colour conditioner formula was applied to a single wetted hair swatch and massaged gently into the hair for 3 minutes using a gloved hand. The colour conditioners were left on the hair for 27 minutes before being rinsed with running water, at 45°C, until the water ran clear (circa 2 minutes). Finally the hair swatches were dried using a 500-watt hair-dryer with gentle combing.

### Colour analysis

Using a Minolta Colorimeter, the base hair swatch colour was measured prior to application of the colour and after the hair had been coloured. The freshly coloured swatches were also visually compared and ranked (blind) for colour intensity, using an untrained panel of volunteers.

### Results

### Colorimeter measurements

| Before colouring: | | | | | | | |
|---|---|---|---|---|---|---|---|
| ① | L 80.06 | ② | L 78.98 | ③ | L 79.12 | **mean** | **L 79.39** |
| | a +0.59 | | a +1.90 | | a +2.10 | | **a +1.53** |
| | b + 17.74 | | b +20.22 | | b +20.94 | | **b + 19.63** |

| After colouring | | | | |
|---|---|---|---|---|
| | **SWATCH I**_{**0**} | **SWATCH J**_{**0**} | **SWATCH K**_{**0**} | **SWATCH L**_{**0**} |
| **Measure 1** | L 51.12 | L 62.06 | L 57.13 | L 58.11 |
| | a +38.61 | a +28.11 | a +33.65 | a +30.89 |
| | b +32.99 | b +28.22 | b +30.58 | b +29.27 |
| **Measure 2** | L 50.05 | L 60.33 | L 55.41 | L 57.94 |
| | a +41.20 | a +31.70 | a +35.33 | a +32.41 |
| | b +34.69 | b +29.65 | b + 31.94 | b + 30.23 |
| **Measure 3** | L 48.78 | L 56.76 | L 52.67 | L 56.23 |
| | a +44.35 | a +35.49 | a +39.17 | a +34.50 |
| | b +36.31 | b +32.17 | b +34.38 | b +31.06 |
| **Mean** | **L 49.98** | **L 59.72** | **L 55.07** | **L 57.43** |
| | **a +41.39** | **a +31.77** | **a + 36.05** | **a + 32.60** |
| | **b +34.66** | **b +30.01** | **b +32.30** | **b +30.19** |

### Visual Assessment (ranking)

| **After colouring** | **Stronger colour** | | | **Less coloured** |
|---|---|---|---|---|
| Volunteer 1 | I | K | L | J |
| Volunteer 2 | I | K | L | J |
| Volunteer 3 | I | K | J | L |
| Volunteer 4 | I | K | L | J |
| Volunteer 5 | I | K | L | J |
| Volunteer 6 | I | K | L | - J |

### Analysis of Data

Comparison of the mean 'L', 'a' and 'b' values of the swatches, shows that the best colour deposition was obtained with the Questamide H containing formulation, followed by Questamix H, then Ceramide II and finally the base colour conditioner. The visual ranking of the hair swatches confirms this result with hair swatches treated with Questamide H, Questamix H showing a stronger colour intensity whilst the base conditioner treated hair was chosen almost unanimously as the poorest.

### Conclusion

The use of the Quest ingredients at the above concentrations produces a stronger colour deposition than the base colour conditioner alone. In particular, Questamide H produces a strong colour deposition that is easily detected by untrained volunteers.

## Claims

1. A method of improving the colouring effects of non-oxidative hair colouring compositions, comprising application to the hair of ceramide, pseudoceramide, glycoceramide and/or neoceramide, in the absence of phytantriol.

2. Use of ceramide, pseudoceramide, glycoceramide and/or neoceramide in a hair care composition for the purpose of improving the colouring effects of a non-oxidative hair dye.

3. The invention according to claim 2, wherein the ceramide, pseudoceramide, glycoceramide and/or neoceramide is present in the hair care composition in an amount in the range 0.01 to 5 % by weight.

4. The invention according to claim 3, wherein the ceramide, pseudoceramide, glycoceramide and/or neoceramide is present in the hair care composition in an amount in the range 0.05 to 1 % by weight.

5. The invention according to any one of the preceding claims, wherein the ceramide, pseudoceramide, glycoceramide and/or neoceramide comprises one or more ceramides or pseudoceramides.

6. The invention according to claim 5, wherein the ceramide comprises ceramide 2.

7. The invention according to claim 5, wherein the pseudoceramide comprises bishydroxyethyl biscetyl malonamide.

## Revendications

1. Méthode pour améliorer les effets colorants des compositions colorantes non-oxydatives pour cheveux, comprenant l'application sur les cheveux de céramide, pseudocéramide, glycocéramide et/ou néocéramide, en absence de phytantriol.

2. Utilisation de céramide, pseudocéramide, glycocéramide et/ou néocéramide dans une composition de soin capillaire dans le but d'améliorer les effets colorants d'un colorant capillaire non-oxydatif.

3. Invention selon la revendication 2, dans laquelle le céramide, pseudocéramide, glycocéramide et/ou néocéramide est présent dans la composition de soin capillaire dans une quantité comprise entre 0,01 et 5 % en poids.

4. Invention selon la revendication 3, dans laquelle le céramide, pseudocéramide, glycocéramide et/ou néocéramide est présent dans la composition de soin capillaire dans une quantité comprise entre 0,05 et 1 % en poids.

5. Invention selon l'une quelconque des revendications précédentes, dans laquelle le céramide, pseudocéramide, glycocéramide et/ou néocéramide comprend un ou plusieurs céramides ou pseudocéramides.

6. Invention selon la revendication 5, dans laquelle le céramide comprend le céramide 2.

7. Invention selon la revendication 5, dans laquelle le pseudocéramide comprend le bishydroxyéthyl biscétyl malonamide.

## Patentansprüche

1. Verfahren zur Verbesserung der Färbeeffekte von nicht-oxidativen Haarfärbezusammensetzungen, das Auftragen von Ceramid, Pseudoceramid, Glycoceramid und/oder Neoceramid in Abwesenheit von Phytantriol auf das Haar umfasst.

2. Verwendung von Ceramid, Pseudoceramid, Glycoceramid und/oder Neoceramid in einer Haarpflegezusammensetzung zum Zwecke der Verbesserung der Färbeeffekte einer nicht-oxidativen Haarfarbe.

3. Erfindung nach Anspruch 2, wobei das Ceramid, Pseudoceramid, Glycoceramid und/oder Neoceramid in der Haarpflegezusammensetzung in einer Menge im Bereich von 0,01 bis 5 Gew.-% vorliegt.

4. Erfindung nach Anspruch 3, wobei das Ceramid, Pseudoceramid, Glycoceramid und/oder Neoceramid in der Haarpflegezusammensetzung in einer Menge im Bereich von 0,05 bis 1 Gew.-% vorliegt.

5. Erfindung nach einem der vorangehenden Ansprüche, wobei das Ceramid, Pseudoceramid, Glycoceramid und/oder Neoceramid ein oder mehrere Ceramide oder Pseudoceramide umfasst.

6. Erfindung nach Anspruch 5, wobei das Ceramid Ceramid 2 umfasst.

7. Erfindung nach Anspruch 5, wobei das Pseudoceramid Bishydroxyethylbiscetylmalonamid umfasst.
